# EUROPEAN PATENT APPLICATION

(11) **EP 4 790 137 A2**
(43) Date of publication of application: **12.08.2026**
(21) Application number: 26189305.1
(22) Date of filing: 28.06.2018
(51) Int. Cl.: C12N 11/00

(54) **LOW-AGGLOMERATION, ENZYME-CONTAINING PARTICLES**

(30) Priority: 30.06.2017 US 201762527396 P
(62) Document number(s) of the earlier application(s) in accordance with Art. 76 EPC: 18743260.4 / 3 645 696
(71) Applicant: Danisco US Inc., Palo Alto, California 94304 (US)
(72) Inventor: DALE, Douglas A., Palo Alto, 94304 (US)
(74) Representative: Mewburn Ellis LLP

(57) **Abstract**

Described are compositions and methods relating to low density enzyme-containing particles for inclusion in cleaning and other low-water compositions. The particles remain in suspension without settling, and release active enzyme upon dilution of the low-water compositions with water.

## Description

### FILED OF THE INVENTION

The present compositions and methods relate to enzyme-containing particles with reduced agglomeration for inclusion in cleaning and other low-water compositions. The particles demonstrate low agglomeration in storage, and release active enzyme upon dilution of the low-water compositions with water.

### BACKGROUND

Enzymes are supplied in both liquid and solid forms for incorporation within products used in a variety of consumer and industrial applications, including laundry and dish cleaning, personal care, textile treatment, pulp and paper production, leather production, food and beverage processing, starch processing, decontamination, oil and gas drilling, production of biofuels, and production (or modification) of biopolymers and other chemicals.

There is a broad need to compartmentalize enzymes or other actives in liquid formulas that contain such incompatible ingredients, so that they are stable during storage, but release quickly upon dilution in application. Many otherwise effective enzymes cannot be utilized because they are unstable in liquid formulations such as detergents.

Aside from present a challenge in terms of stability, enzymes are immunogenic molecules and can present problems relating to exposure and sensitization. In some cases, the maximum amount of enzymes that can be added to a liquid cleaning formulation is determined by exposure risk, as opposed to performance or economics.

Enzymes can be provided in granular form in liquid detergent but granules invariably settle in liquid formulations such as detergents, resulting in non-uniform distribution of enzymes as well as the unappealing appearance of settled granules. Accordingly, there is a need for improved ways to compartmentalize enzymes in liquid formulations, such that they remain stable, retains catalytic potential until use in an application in which enzyme activity is desired, and remain uniformly suspended, without agglomerating, in a liquid for prolonged periods of time.

### BRIEF SUMMARY OF THE INVENTION

The invention provides low-density particles for isolating and stabilizing enzymes in aqueous compositions, and methods of use, thereof. Aspects and embodiments of the invention are described in the following numbered paragraphs.
1. In one aspect, particles capable of isolating and stabilizing enzymes in a liquid composition without agglomerating in manufacturing and/or storage are provided, comprising: (a) a core, including an active component, and/or a core having a first coated layer comprising an active component immediately deposited upon the core; and (b) an outer-most coated layer comprising a hydrophobic, water-insoluble, water-disintegrating-material having an amount of water solubility of less than about 1 mg/mL in water at 25°C; wherein the coated layer in (b) fully disintegrates within about 5 minutes when the liquid composition is diluted 1:1 with water at 25°, allowing the dissolution of the enzyme and/or active component into the diluted liquid composition, and wherein the particles exhibit reduced aggregation in the liquid composition compared to otherwise identical particles comprising a third coated layer comprising a water-soluble polymer having a solubility of greater than about 1 mg/mL in water at 25°C.
2. In some embodiments, the particles of paragraph 1 further comprise, between (a) and (b), at least one additional layer comprising a water-soluble polymer and an active ingredient.
3. In some embodiments, the particles of paragraph 1 further comprise, between (a) and (b) at least one additional layer comprising a water-soluble polymer lacking an active ingredient.
4. In some embodiments of the particles of paragraphs 1 or 2, the core lacks an active component.
5. In some embodiments of the particles of paragraphs 1 or 3, the core includes an active component.
6. In some embodiments of the particles of any of the preceding paragraphs, the outer-most coating disintegrates within 5 minutes, within 4 minutes, within 3 minutes, within 2 minutes, within 1 minute, within 30 seconds, or even within 15 seconds after a liquid composition containing the particles is contacted with at least one additional volume of water at 25°C.
7. In some embodiments of the particles of any of the preceding paragraphs, the outer-most coating represents less than 8%, less than 7%, less than 6%, or even less than 5% of the overall weight of the particle.
8. In some embodiments of the particles of any of the preceding paragraphs, the outer-most consists essentially of, or consists of, a hydrophobic, water-insoluble, water-disintegrating-material having an amount of water solubility of less than about 1 mg/mL in water at 25°C.
9. In some embodiments of the particles of any of the preceding paragraphs, the core has a density defined by the equations:${ρ}_{c} \leq \left({ρ}_{f} + {{31250 / D}_{p}}^{2}\right) ∗ {x}_{c} / {\left({D}_{c}{/ D}_{ρ}\right)}^{\left(1/3\right)}$ and${ρ}_{c} \leq \left({ρ}_{f} - {{31250 / D}_{p}}^{2}\right) ∗ {x}_{c} / {\left({D}_{c}{/ D}_{ρ}\right)}^{\left(1/3\right)}$ wherein ρ*_{c}* is the density of the core in in g/cm³, *ρ_{f}* is the mass density of the liquid composition in g/cm³, x*_{c}* is the mass fraction of the core in the particle, D*_{c}* is the diameter of the core in µM, and D*ₚ* is the diameter of the particle in µM.
10. In some embodiments, the particles of any of the preceding paragraphs have an overall true density of less than 1.6 mg/mL, less than 1.4 mg/mL, or even less than 1.2 mg/mL.
11. In another aspect, a method for reducing the agglomeration of particles in manufacturing and/or storage is provided, comprising coating the particles in an outer-most layer comprising a hydrophobic, water-insoluble, water-disintegrating-material having an amount of water solubility of less than about 1 mg/mL in water at 25°C.
12. In some embodiments of the method of paragraph 11, the outer-most consists essentially of, or consists of, a hydrophobic, water-insoluble, water-disintegrating-material having an amount of water solubility of less than about 1 mg/mL in water at 25°C.
13. In some embodiments of the method of paragraph 11 or 12, the outer-most coating disintegrates within 5 minutes, within 4 minutes, within 3 minutes, within 2 minutes, within 1 minute, within 30 seconds, or even within 15 seconds after a liquid composition containing the particles is contacted with at least one additional volume of water at 25°C.
14. In some embodiments of the method of any of paragraphs 11-13, the outer-most coating represents less than 8%, less than 7%, less than 6%, or even less than 5% of the overall weight of the particle.

These and other aspects and embodiments of the compositions and methods are described, below.

### DETAILED DESCRIPTION

### I. Definitions and abbreviations

Unless defined otherwise herein, all technical and scientific terms used herein have the same meaning as commonly understood by one of ordinary skill in the art to which this invention pertains. Although any methods and materials similar or equivalent to those described herein find use in the practice of the present invention, the preferred methods and materials are described herein. Accordingly, the terms defined immediately below are more fully described by reference to the Specification as a whole. Also, as used herein, the singular terms "a," "an," and "the" include the plural reference unless the context clearly indicates otherwise. Unless otherwise indicated, nucleic acids are written left to right in 5' to 3' orientation: amino acid sequences are written left to right in amino to carboxy orientation, respectively. It is to be understood that this invention is not limited to the particular methodology, protocols, and reagents described, as these may vary, depending upon the context they are used by those of skill in the art.

It is intended that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

As used herein, the term "water soluble polymer" refers to a polymer that is soluble in water in in an amount of at least 1 mg/ml. As used herein, an "aqueous medium" or "aqueous solution" is a solution and/or suspension in which the solvent is primarily water *(i.e.,* the solvent is at least 50% water, at least 60% water, at least 70% water, at least 80% water, or even at least 90% water). The aqueous medium may include any number of dissolved or suspended components, including but not limited to surfactants, salts, buffers, stabilizers, complexing agents, chelating agents, builders, metal ions, additional enzymes and substrates, and the like. Exemplary aqueous media are laundry and dishwashing wash liquors. Materials such as textiles, fabrics, dishes, kitchenware, and other materials may also be present in or in contact with the aqueous medium.

As used herein, the term "water-insoluble material" refers to a material that is not soluble in water even when mixed, such as a material with a solubility of less than 1 mg/ml in water at 25°C.

As used herein, the term "hydrophobic" refers to a material that is repelled from (or repels) water. That is, there is no attractive forces between the material and water.

As used herein, the term hydrophilic-lipophilic balance (HLB) refers to the empirical expression of relationship of hydrophilic and hydrophobic groups of a surfactant.

As used herein, the term "disintegrating material" refers to a material that is not soluble in water, but has the ability to break down from larger particles into smaller particles that are capable of being suspended in water when mixed.

As used herein, the term "agglomeration" refers to the phenomena wherein individual particles come together to form groups or clusters of multiple particles. The association between the particles can either be a loose association or a tight association, including by covalent bonds formed between the particles.

As used herein, the term "low-water," with reference to a liquid laundry detergent composition, indicates that the detergent composition contains about 5% to 20% water (w/w).

As used herein, the term "substantially non-aqueous," with reference to a liquid laundry detergent composition, indicates that the detergent composition contains about 2-5% water (w/w).

As used herein, a "non-aqueous" solution contains less than about 2% water (w/w).

As used herein, where a component is "provided in" a specified form (e.g., non-aqueous, very low water, solid, and the like), this form refers to the final form as the component exists in the unit-dose package, not the form in which it may be added to another component that is then added to the unit-dose package.

As used herein, the phrase "insufficient to substantially dissolve water-soluble packaging" means that a subject liquid does not dissolve more than 5% of a water-soluble material over a period of six months at room temperature (*i.e*., 25°C).

As used herein, the term "bounded" with reference to the contents of water-soluble packaging means the specified contents, whether liquid, solid, or a combination, thereof, are physically contained in a compartment, at least a portion of which is defined by water-soluble material. In some cases, the contents are fully bounded by water-soluble material, meaning that the entire compartment is defined by the water-soluble material, as in the case of a pouch made of water-soluble material. In some cases, the contents are only partially bounded by water-soluble material, meaning that only a portion of the compartment is defined by the water soluble material, and the remainder is defined by water-insoluble material, as in the case of a cup or dish covered by a lid made of water-soluble material.

As used herein, the terms "suspended" and "dispersed" refer to the distribution of one component in another, for example, the distribution of a solid form of acyl substrate in water-soluble material.

As used herein, "cold" water is water having a temperature between freezing and about 25°C.

As used herein, "room temperature" is 25°C.

As used herein, "warm" water is water having a temperature between about 26°C and about 37°C.

As used herein, "hot" water is water having a temperature between about 37°C and boiling.

As used herein, a "low" pH is a pH of less than about 7.

As used herein, a "high" pH is a pH of greater than about 7.

As used herein, the term "contacting," means bringing into physical contact, such as by placing a unit-dose package in an aqueous solution.

As used herein, a "solid" form of a chemical component refers to a powder, crystals, granules, aggregates, paste or wax thereof.

As used herein, a "liquid" form of a chemical component refers to a liquid, gel, or slurry.

As used herein, "true density" refers to the mass of a particle divided by its volume, excluding open pores and closed pores.

As used herein, the term "spray drying" refers to a method of producing a dry powder from a liquid or slurry by rapidly drying with a hot gas, as known in the art and discussed for example in US Patent 5,423,997 and WO2008/088751A2.

As used herein "d50" refers to the size of the particles measured where 50% are above or below the mid-point within the population measured.

As used herein, the term "UFC Solids" refers to ultrafiltrate concentrate from a fermenter/bioreactor, and is synonymous with enzyme concentrate solids.

As used herein, "cleaning compositions" and "cleaning formulations" refer to compositions that may be used for the removal of undesired compounds from items to be cleaned, such as fabric, dishes, contact lenses, other solid substrates, hair (shampoos), skin (soaps and creams), teeth (mouthwashes, toothpastes) etc. The term encompasses any materials/compounds selected for the particular type of cleaning composition desired. The specific selection of cleaning composition materials are readily made by considering the surface, item or fabric to be cleaned, and the desired form of the composition for the cleaning conditions during use.

The terms further refer to any composition that is suited for cleaning, bleaching, disinfecting, and/or sterilizing any object and/or surface. It is intended that the terms include, but are not limited to detergent compositions (e.g., laundry detergents and fine fabric detergents; hard surface cleaning formulations, such as for glass, wood, ceramic and metal counter tops and windows; carpet cleaners; oven cleaners; fabric fresheners; fabric softeners; and textile and laundry pre-spotters, as well as dish detergents).

As used herein, the terms "detergent composition" and "detergent formulation" are used in reference to mixtures which are intended for use in a wash medium for the cleaning of soiled objects. In some preferred embodiments, the term is used in reference to laundering fabrics and/or garments (e.g., "laundry detergents"). In alternative embodiments, the term refers to other detergents, such as those used to clean dishes, cutlery, etc. (e.g., "dishwashing detergents").

As used herein, the term "nonionic surfactant" refers to a surfactant molecule with a non-electrically charged polar group.

As used herein, the term "anionic surfactant" refers to a surfactant molecule with a negatively charged polar group at the pH of the composition or the application of use. Salts used to complex or neutralize the surfactant, *e.g.*, forming the monoethanolamine (MEA) salt of linear alkylbenzene sulfonate (LAS) are included I accounting herein for the mass or concentration of anionic surfactant.

As used herein, the phrase "detergent stability" refers to the stability of a detergent composition. In some embodiments, the stability is assessed during the use of the detergent, while in other embodiments, the term refers to the stability of a detergent composition during storage.

As used herein the term "hard surface cleaning composition" refers to detergent compositions for cleaning hard surfaces such as floors, walls, tile, bath and kitchen fixtures, and the like.

As used herein, "non-fabric cleaning compositions" encompass hard surface cleaning compositions, dishwashing compositions, personal care cleaning compositions *(e.g.,* oral cleaning compositions, denture cleaning compositions, personal cleansing compositions, etc.), and compositions suitable for use in the pulp and paper industry.

As used herein, "personal care products" means products used in the cleaning, bleaching and/or disinfecting of hair, skin, scalp, and teeth, including, but not limited to shampoos, body lotions, shower gels, topical moisturizers, toothpaste, and/or other topical cleansers. In some particularly preferred embodiments, these products are utilized on humans, while in other embodiments, these products find use with non-human animals (e.g., in veterinary applications).

"Water miscible" as used herein refers to a liquid forming a single thermodynamic liquid phase or isotropic phase upon mixing with water, at a specified ratio of water to the liquid.

A "suspension" or "dispersion" as used herein refers to a two phase system wherein a discontinuous solid phase is dispersed within a continuous liquid phase. The solid phase can consist of very fine particles or larger granules, and the particles or granules can have a wide variety of shapes, morphologies and structures. For example, the solids can be spray dried particles as small as 1 micron in diameter or larger core-shell granules between 100 and 1,000 microns in diameter.

A "suspension aid" as used herein refers to a material added to a liquid composition to prevent or reduce sedimentation or floating of suspended particles. Suspension aids typically work by increasing either the viscosity or the yield stress of a carrier liquid. Fluids with a significant yield stress will flow only when stress is applied which is greater than the yield stress, and thus exhibit shear-thinning or thixotropic behavior. Effective suspension agents typically act by forming a reversible network of particles or fibers bridged by weak forces. Examples of suspending agents include, but are not limited to, xanthan gum and microfibrous cellulose, *e.g.*, Cellulon (CP Kelco, San Diego, CA).

The following abbreviations may be used in the description. Definitions are also provided as needed throughout the description.
- °C: degrees Centigrade
- AU: activity units
- CaCl₂: calcium chloride
- Cm: centimeter
- cm³: cubic centimeters
- D(0.5): median particle size where 50% of the particles are at or below the specified diameter
- D(0.9): median particle size where 90% of the particles are at or below the specified diameter
- dH₂O or DI: deionized water
- eq.: equivalents
- ETOH: ethanol
- g or gm: Grams (note, below)
- H₂O: water
- hr: hour
- M: molar
- melting temperature: melting temperature
- mg: milligrams
- min: minute
- mL and ml: milliliters
- mm: millimeters
- mM: millimolar
- MW: molecular weight
- N: normal
- Na₂SO₄: sodium sulfate
- NaOH: sodium hydroxide
- nm: nanometer
- PE: polyethylene
- PEG: polyethyleneglycol
- ppm: parts per million
- PVA: poly(vinyl alcohol)
- PVP: poly(vinylpyrrolidone)
- sec: seconds
- TiO₂: titanium dioxide
- U: units
- v/v: volume/volume
- w/v: weight/volume
- w/w: weight/weight
- wt%: weight percent
- µg: micrograms
- µL and µl: microliters
- µm: micrometer
- µM: micromolar

### II. Particles with hydrophobic or water-insoluble, water-disintegrating coatings

It is often desirable to incorporate particles with active agents into low-water liquid detergents in order to provide cleaning or other benefits. Unfortunately, conventional particles having outer surfaces made from materials with water-soluble or hydrophilic properties have been shown to agglomerate during and/or following incorporation into the low water detergents, such as during manufacturing, mixing, handling, transportation and/or storage.

The present materials and methods overcome this undesirable agglomeration phenomenon by using hydrophobic and/or water-insoluble materials on the outer surface of the particles to prevent agglomeration from occurring, which materials readily disintegrate when the low water detergent is diluted into a wash liquid.

Generally, the particles include (i) a core, (ii) at least one enzyme and/or other active component-containing layer, (iii) one or more additional layers and (iv) an outer-most coating with hydrophobic and/or water-insoluble properties that will rapidly disintegrate when diluted 1:1 in water. These components are described in greater detail.

### A. Hydrophobic or water-insoluble and water-disintegrating surface coatings

A key feature of the present compositions and methods, is a particle having an outer-most coating with hydrophobic and/or water-insoluble properties to prevent the particles from agglomerating during manufacture, handling, transportation and/or storage, but which readily disintegrates when diluted into a wash liquor.

Exemplary materials that have the necessary hydrophobic and/or water-insoluble properties but which are readily disintegrating upon dilution include, but are not limited to, natural waxes, such as carnauba, beeswax, palmitic wax, candelilla wax, synthetic waxes such as paraffin wax and microcrystalline wax, low HLB surfactants such as those with values below HLB=6, hydrophobically modified polyvinyl alcohol, hydrophobically modified starch such as those modified with fatty acid side chains, hydrophobically modified cellulosic polymers.

Ideally, The melting point of the outer coting materials should be high enough to remain solid during processing and storage. Accordingly, the melting temperature should be above 40°C, above 45°C, above 50°C, above 55°C, or even above 60°C, depending on the process conditions and application.

The coating should disintegrate within 5 minutes, within 4 minutes, within 3 minutes, within 2 minutes, within 1 minute, within 30 seconds, or even within 15 seconds after the low-water liquid composition containing the particles is contacted with at least one additional volume of water at 25°C.

The outer coating composition need only to be incorporated at level sufficient to impart the desired surface properties to avoid agglomerations and to allow for rapid disintegration upon dilution in wash liquor. Accordingly, the outer coating should be as thin as possible. In some embodiments, the percent weight (wt/wt%) of the outer-most coating relative to the particle as a whole should be less than 8%, less than 7%, less than 6%, or even less than 5%.

### B. Coating containing enzymes and other actives

The cores (to be described, *infra*) are coated with and/or may optionally contain one or more of a wide variety of enzymes or other actives. While the present description is focused on enzymes, it will be apparent that a myriad of other active components can be provided in a low-water composition using the same particles.

Exemplary enzymes include acyl transferases, α-amylases, β-amylases, α-galactosidases, arabinosidases, aryl esterases, β-galactosidases, carrageenases, catalases, cellobiohydrolases, cellulases, chondroitinases, cutinases, endo-β-1, 4-glucanases, endo-beta-mannanases, esterases, exo-mannanases, galactanases, glucoamylases, hemicellulases, hyaluronidases, keratinases, laccases, lactases, ligninases, lipases, lipoxygenases, mannanases, oxidases, oxidoreductases, pectate lyases, pectin acetyl esterases, pectinases, pentosanases, perhydrolases, peroxidases, peroxygenases, phenoloxidases, phosphatases, phospholipases, phytases, polygalacturonases, proteases, pullulanases, reductases, rhamnogalacturonases, β-glucanases, tannases, transglutaminases, xylan acetyl-esterases, xylanases, xyloglucanases, xylosidases, metalloproteases, additional serine proteases, and combinations, thereof.

Examples of suitable proteases include but are not limited to subtilisins, such as those derived from *Bacillus (e.g.,* subtilisin, lentus, amyloliquefaciens, subtilisin Carlsberg, subtilisin 309, subtilisin 147 and subtilisin 168), including variants as described in, *e.g.*, U.S. Pat. Nos. RE 34,606, 5,955,340, 5,700,676, 6,312,936, and 6,482,628, all of which are incorporated herein by reference. Additional proteases include trypsin (e.g., of porcine or bovine origin) and the *Fusarium* protease described in WO 89/06270. In some embodiments the protease is one or more of MAXATASE^{®}, MAXACAL^{™}, MAXAPEM^{™}, OPTICLEAN^{®}, OPTIMASE^{®}, PROPERASE^{®}, PURAFECT^{®}, PURAFECT^{®} OXP, PURAMAX^{™}, EXCELLASE^{™}, and PURAFAST^{™} (Genencor); ALCALASE^{®}, SAVINASE^{®}, PRIMASE^{®}, DURAZYM^{™}, POLARZYME^{®}, OVOZYME^{®}, KANNASE^{®}, LIQUANASE^{®}, NEUTRASE^{®}, RELASE^{®} and ESPERASE^{®} (Novozymes); BLAP^{™} and BLAP^{™} variants (Henkel Kommanditgesellschaft auf Aktien, Duesseldorf, Germany), and KAP *(B. alkalophilus* subtilisin; Kao Corp., Tokyo, Japan). Additional proteases are described in WO95/23221, WO 92/21760, WO 09/149200, WO 09/149144, WO 09/149145, WO 11/072099, WO 10/056640, WO 10/056653, WO 11/140364, WO 12/151534, U.S. Pat. Publ. No. 2008/0090747, and U.S. Pat. Nos. 5,801,039, 5,340,735, 5,500,364, 5,855,625, US RE 34,606, 5,955,340, 5,700,676, 6,312,936, and 6,482,628.

Suitable proteases include neutral metalloproteases including those described in WO 07/044993 and WO 09/058661. Other exemplary metalloproteases include nprE, the recombinant form of neutral metalloprotease expressed in *Bacillus subtilis* (see *e.g.,* WO 07/044993), and PMN, the purified neutral metalloprotease from *Bacillus amyloliquefacients.*

Suitable lipases include, but are not limited to *Humicola lanuginosa* lipase (see *e.g.,* EP 258 068, and EP 305 216), *Rhizomucor miehei* lipase (See *e.g.,* EP 238 023), Candida lipase, such as C. *antarctica* lipase *(e.g.,* the C. *antarctica* lipase A or B; See *e.g.,* EP 214 761), Pseudomonas lipases such as *P. alcaligenes* lipase and *P. pseudoalcaligenes* lipase (See *e.g.,* EP 218 272), *P. cepacia* lipase (See *e.g.,* EP 331 376), *P. stutzeri* lipase (See *e.g.,* GB 1,372,034), *P. fluorescens* lipase, *Bacillus* lipase *(e.g., B. subtilis* lipase (Dartois et al. (1993) Biochem. Biophys. Acta 1131:253-260); *B. stearothermophilus* lipase (see *e.g.,* JP 64/744992); and *B. pumilus* lipase (see *e.g.,* WO 91/16422)).

Additional suitable lipases include *Penicillium camembertii* lipase (Yamaguchi et al. (1991) Gene 103:61-67), *Geotricum candidum* lipase (See, Schimada et al. (1989) J. Biochem. 106:383-388), and various Rhizopus lipases such as *R. delemar* lipase (Hass et al. (1991) Gene 109:117-113), a *R. niveus* lipase (Kugimiya et al. (1992) Biosci. Biotech. Biochem. 56:716-719) and *R. oryzae* lipase. Additional lipases are the cutinase derived from *Pseudomonas mendocina* (See, WO 88/09367), and the cutinase derived from *Fusarium solani pisi* (WO 90/09446). Various lipases are described in WO 11/111143, WO 10/065455, WO 11/084412, WO 10/107560, WO 11/084417, WO 11/084599, WO 11/150157, and WO 13/033318. In some embodiments the protease is one or more of M1 LIPASE^{™}, LUMA FAST^{™}, and LIPOMAX^{™} (Genencor); LIPEX^{®}, LIPOLASE^{®} and LIPOLASE^{®} ULTRA (Novozymes); and LIPASE P^{™} "Amano" (Amano Pharmaceutical Co. Ltd., Japan).

Suitable amylases include, but are not limited to those of bacterial or fungal origin, or even mammalian origin. Numerous suitable are described in WO9510603, WO9526397, WO9623874, WO9623873, WO9741213, WO9919467, WO0060060, WO0029560, WO9923211, WO9946399, WO0060058, WO0060059, WO9942567, WO0114532, WO02092797, WO0166712, WO0188107, WO0196537, WO0210355, WO9402597, WO0231124, WO9943793, WO9943794, WO2004113551, WO2005001064, WO2005003311, WO0164852, WO2006063594, WO2006066594, WO2006066596, WO2006012899, WO2008092919, WO2008000825, WO2005018336, WO2005066338, WO2009140504, WO2005019443, WO2010091221, WO2010088447, WO0134784, WO2006012902, WO2006031554, WO2006136161, WO2008101894, WO2010059413, WO2011098531, WO2011080352, WO2011080353, WO2011080354, WO2011082425, WO2011082429, WO2011076123, WO2011087836, WO2011076897, WO94183314, WO9535382, WO9909183, WO9826078, WO9902702, WO9743424, WO9929876, WO9100353, WO9605295, WO9630481, WO9710342, WO2008088493, WO2009149419, WO2009061381, WO2009100102, WO2010104675, WO2010117511, WO2010115021, WO2013184577, WO9418314, WO2008112459, WO2013063460, WO10115028, WO2009061380, WO2009100102, WO2014099523, WO2015077126A1, WO2013184577, WO2014164777, PCT/US12/70334, PCT/US13/74282, PCT/CN2013/077294, PCT/CN2013/077134, PCT/CN2013/077137, PCT/CN2013/077142, PCT/CN2012/087135, PCT/US 12/62209, PCT/CN2013/084808, PCT/CN2013/084809, and PCT/US14/23458. Commercially available amylases include, but are not limited to one or more of DURAMYL^{®}, TERMAMYL^{®}, FUNGAMYL^{®}, STAINZYME^{®}, STAINZYME PLUS^{®}, STAINZYME ULTRA^{®}, and BAN^{™} (Novozymes), as well as POWERASE^{™}, RAPIDASE^{®} and MAXAMYL^{®} P, PREFERENZ^{®} S100, PREFERENZ^{®} S110, and PREFERENZ^{®} S1000 (Genencor).

Suitable cellulases include but are not limited to those having color care benefits (see *e.g.,* EP 0 495 257). Examples include *Humicola insolens* cellulases (See *e.g.,* U.S. Pat. No. 4,435,307) and commercially available cellulases such as CELLUZYME^{®}, CAREZYME^{®} (Novozymes), and KAC-500(B)^{™} (Kao Corporation), and Primafast^{®} GOLD (DuPont). In some embodiments, cellulases are incorporated as portions or fragments of mature wild-type or variant cellulases, wherein a portion of the N-terminus is deleted (See e.g., U.S. Pat. No. 5,874,276). Additional suitable cellulases include those found in WO2005054475, WO2005056787, U.S. Pat. No. 7,449,318, and U.S. Pat. No. 7,833,773.

Suitable mannanases are described in U.S. Pat. Nos. 6,566,114, 6,602,842, 5, 476, and 775, 6,440,991, and U.S. Patent Application Number 61/739267, all of which are incorporated herein by reference). Commercially available include, but are not limited to MANNASTAR^{®}, PURABRITE^{™}, and MANNAWAY^{®}.

In some embodiments, peroxidases are used in combination with hydrogen peroxide or a source thereof *(e.g.,* a percarbonate, perborate or persulfate) in the compositions of the present teachings. In some alternative embodiments, oxidases are used in combination with oxygen. Both types of enzymes are used for "solution bleaching" *(i.e.,* to prevent transfer of a textile dye from a dyed fabric to another fabric when the fabrics are washed together in a wash liquor), preferably together with an enhancing agent (See *e.g.*, WO 94/12621 and WO 95/01426). Suitable peroxidases/oxidases include, but are not limited to those of plant, bacterial or fungal origin. Chemically or genetically modified mutants are included in some embodiments.

Suitable perhydrolases include the enzyme from *Mycobacterium smegmatis.* This enzyme, its enzymatic properties, its structure, and numerous variants and homologs, thereof, are described in detail in International Patent Application Publications WO 05/056782A and WO 08/063400A, and U.S. Patent Publications US2008145353 and US2007167344, which are incorporated by reference. In some embodiments, *the Mycobacterium smegmatis* perhydrolase, or homolog, includes the S54V substitution.

Other suitable perhydrolases include members of the carbohydrate family esterase family 7 (CE-7 family) described in, *e.g.*, WO2007/070609 and U.S. Patent Application Publication Nos. 2008/0176299, 2008/176783, and 2009/0005590. Members of the CE-7 family include cephalosporin C deacetylases (CAHs; E.C. 3.1.1.41) and acetyl xylan esterases (AXEs; E.C. 3.1.1.72). Members of the CE-7 esterase family share a conserved signature motif (Vincent et al., J. Mol. Biol., 330:593-606 (2003)).

Other suitable perhydrolase enzymes include those from *Sinorhizobium meliloti, Mesorhizobium loti, Moraxella bovis, Agrobacterium tumefaciens,* or Prosthecobacter *dejongeii* (WO2005056782), *Pseudomonas mendocina* (U.S. Patent No. 5,389,536), or *Pseudomonas putida* (U.S. Patent Nos. 5,030,240 and 5,108,457).

The enzymes may be crystalized, precipitated, spray dried, lyophilized, and/or compressed and provided in dry form, or resuspended liquid form, thereof. The enzymes may be provided as an ultrafiltration concentrate. They may be purified to a preselected level.

The cores may further be coated with and/or contain one or more additional components, such as bleach catalysts, stabilizing systems, chelants, optical brighteners, soil release polymers, dye transfer agents, dispersants, suds suppressors, dyes, perfumes, colorants, filler salts, photoactivators, fluorescers, fabric conditioners, hydrolyzable surfactants, preservatives, anti-oxidants, anti-shrinkage agents, anti-wrinkle agents, germicides, fungicides, color speckles, silvercare, anti-tarnish and/or anti-corrosion agents, alkalinity sources, solubilizing agents, carriers, processing aids, pigments, and pH control agents, surfactants, builders, dye transfer inhibiting agents, deposition aids, catalytic materials, bleach activators, bleach boosters, hydrogen peroxide, sources of hydrogen peroxide, preformed peracids, polymeric dispersing agents, clay soil removal/antiredeposition agents, brighteners, structure elasticizing agents, fabric softeners, hydrotropes, processing aids and/or pigments. Suitable examples of such other adjuncts and levels of use are found in U.S. Patent Nos. 5,576,282, 6,306,812, 6,326,348, 6,610,642, 6,605,458, 5,705,464, 5,710,115, 5,698,504, 5,695,679, 5,686,014 and 5,646,101 all of which are incorporated herein by reference. Representative detergent formulations useful for the present invention include the detergent formulations found in WO2013063460, WO2003010266, WO2006002755, WO2006088535, and US20110263475, all of which are hereby incorporated by reference. Such adjuvants can be included in the core, the enzyme layer, or the polymer coating, so long as they do not adversely affect the described desired properties of the particles.

### C. Additional coatings

Depending on the particular embodiments of the present particles and methods, there may be included at least one non-aqueous, water-soluble coating applied to the core, or coated core, to protect the enzyme and/or other active component layer from water present in the low-water liquid compositions in which the particles are intended to be suspended. The coating should be non-toxic and biodegradable. The solubility of the coating in water should be greater than 1, greater than 2, greater than 3, greater than 4, greater than 5, greater than 6, greater than 7, greater than 8, greater than 9, or even greater than 10 mg/mL at 25°C. The coating should dissolve within 5 minutes, within 4 minutes, within 3 minutes, within 2 minutes, within 1 minute, within 30 seconds, or even within 15 seconds when the low-water liquid composition in which they are suspended is diluted with at least one volume of water.

Exemplary materials are linear or branched polymers having a molecular weight such that the polymer (or mixture of different polymers) is/are solid at room temperature).. Specific exemplary materials include but are not limited to synthetic polymers, such as polyvinyl alcohol (PVA), polyvinyl acetate, polyvinyl pyrrolidone (PVP), polyethylene glycol (PEG), polyethylene oxide (PEO), poly acrylic acid, poly methacrylic acid, pyrrolidone carboxylic acid, polystyrene sulfonates, and polyelectrolytes; fatty acids, such as stearic acid, oleic acid, myristic acid, and palmitic acid: gums, such as acacia, guar, xanthan, agarose, karaya, tragacanth, and locust bean: cellulosic materials, such as hydroxy propyl cellulose, hydroxypropyl methylcellulose, cellulose acetate butyrate, cellulose acetate phthalate, carboxy methyl cellulose (CMC), methyl cellulose, and hydroxy ethyl cellulose; and other materials, such as cucurbuturil, polyethylimine, quaternary polyamine, carrageenan, pectins, chitosan, polysacharrides, poloxamers, polyanhydrides, polyhydroxyalkanoates, gluten, gelatin, sodium alginate, carrageenan, starch, dextrins, , and; and mixtures, thereof.

### D. Cores

In some embodiments, the core of the present particles, which feature an outer, hydrophobic or water-insoluble, water-disintegrating, outer-coating, is not critical to the present compositions and methods, and may be of a conventional nature. Commonly used material are salts and sugars and other relatively inexpensive, water soluble materials. The core may be inert, or may feature active ingredients. In other embodiments, the core may include some, or even all the active agents, such as enzymes, mentioned, above.

In particular embodiments, the core is selected such that the particles have an overall particle density close to the density of the low-water liquid composition in which they are suspended or intended-to-be suspended. This distinguishes further distinguishes the present particles from conventional particles, which typically have a higher density, and tend to settle out of suspension.

The low density of the particles may be achieved by one of two approaches, or a combination of both. A first approach is to use low-density cores. Various materials for making low density cores are described, below, and several are exemplified, herein. A second approach is to use more conventional medium-to-high-density cores, in combination with a density modifier to reduce the overall density of the particle. These approaches can readily be combined such that the selection of the core material and the use of a density modifier both contribute to the overall low density of the particle. Alternatively, a density modifier can be used to fine tune the overall density of a particle based on a preselected core particle, as in the case of tailoring standardized particles for use in different low water compositions having different densities.

### 1. Cores made from low-density materials

The core of the particle may be made from one or more non-toxic and biodegradable materials. Preferably, the cores dissolve or disperse in water. As described, above, the cores may have a density similar to that of the low-water composition in which they are intended to be suspended liquid, such that they remain uniformly suspended in the carrier liquid without substantial settling. Most aqueous liquids have a density between 1.0 g/cm³ and 1.3 g/cm³, depending on the dissolved solutes, and the density of the core should be within 0.5 g/cm³, 0.4 g/cm³, 0.3 g/cm³, 0.2 g/cm³, or even 0.1 g/cm³ of the density of the liquid.

The desired density of the cores depends on the relative size of the cores compared to the overall size of the particles. A larger core represents a larger portion of the overall particle, making its density more critical. A smaller core may represent only a small portion of the overall particle, making its density less critical. The desired density of the core can be selected based on Stoke's law for calculating the settling velocity of a particle in a viscous medium: ${v}_{s} = \frac{2}{9} \frac{\left({ρ}_{p}-{ρ}_{f}\right)}{μ} g {R}^{2}$

In the equation, above, *νₛ* is the particle's settling velocity (e.g., m/s), which is vertically downwards if *ρₚ > ρ_{f}* and vertically upwards if *ρₚ < ρ_{f} ),* g is gravitational acceleration (m/s²), *ρₚ* is the mass density of the particle *(e.g.,* kg/m³), *p_{f}* is the mass density of the fluid (kg/m³), *µ* is the dynamic viscosity *(e.g.,* kg/m*s) of the water liquid in which the particle is suspended, and *R* is the particle radius (m). For convenience in view of the small size of the subject particles, other units may be used, for example, particle diameter and radius are preferably expressed in µm.

For a given liquid composition, the viscosity (*µ*) is held constant, so to maintain a constant settling viscosity the required density difference scales with the square of the particle radius or diameter and the other coefficients can be ignored since they cancel out of any ratio. An exemplary particle has a diameter of 250 µm and a radius of 125 µm. For this particle, the absolute value of the density difference between particle density (ρ*ₚ*) and fluid density (ρ*_{f}*), *i.e.,* (ρ*ₚ-*ρ*_{f}* or Δρ*_{pf}*) should be no more than 0.5 g/cm³, so any particle that is larger or smaller than 250 µm diameter is acceptable as long as the settling rate (*νₛ*) does not increase. With the liquid medium viscosity fixed, any particle will have the same *νₛ* when: $\left(\left|{Δρ}_{pf}\right|*{{D}_{p}}^{2}\right) = \left(0.5\right) * {\left(250\right)}^{2}$ where D*ₚ* is the overall diameter of the particle. Such a particle will not settle (or rise) faster than *νₛ* when for the maximum density the difference is given by: $\left|{Δρ}_{pf}\right| < \left(0.5\right) * {\left(250\right)}^{2} / {{D}_{p}}^{2}$ or $\left|{Δρ}_{pf}\right| \leq 31250 / {{D}_{p}}^{2}$

Expressed in another way: ${ρ}_{p} \leq {ρ}_{f} + 31250 / {{D}_{p}}^{2} , to avoid settling$ ${ρ}_{p} \geq {ρ}_{f} - 31250 / {{D}_{p}}^{2} , to avoid floating$

Using the latter formula, the maximum density difference (|Δρ*_{pf}*|) required as a function of overall particle diameters (D*ₚ*) can be calculated, as shown in Table 1:

**Table 1. Maximum density differences for different overall particle diameters**

| D*ₚ* (µm) | \|Δρ*_{pf}*\| max (g/cm³) |
|---|---|
| 50 | 12.5 |
| 100 | 3.13 |
| *150* | 1.39 |
| 200 | 0.78 |
| 250 | 0.50 |
| 300 | 0.35 |
| 350 | 0.26 |
| 400 | 0.20 |
| 500 | 0.13 |
| 600 | 0.09 |
| 700 | 0.06 |
| 800 | 0.05 |
| 900 | 0.04 |
| 1000 | 0.03 |

The above relationship can also be extended to define the constraints on the density of the core (ρ*_{c}*) within the overall particle (ρ*ₚ*). The density of the core can be related to the density of the overall particle according to the relationship: ${ρ}_{c} / {ρ}_{p} = \left({m}_{c}/{v}_{c}\right) / \left({m}_{p}/{v}_{p}\right)$ where m*_{c}* and m*ₚ* represent the mass of the core and mass of the overall particle, respectively, and v*ₚ* and v*_{c}* represent the respective volumes of the overall particle and the core. Rearranging: ${ρ}_{c} = {ρ}_{c} * {m}_{c} / {m}_{p} * \left({v}_{p}/{v}_{p}\right)$

Expressing the volumes in terms of diameters of the core (D*_{c}*) and particle (D*ₚ*) and representing the mass fraction of the core as x*_{c}*, we obtain: ${ρ}_{c} = {ρ}_{p} * {x}_{c} / {\left({D}_{c}/{D}_{p}\right)}^{\left(1/3\right)}$ or we can show the particle density in terms of core density: ${ρ}_{p} = {ρ}_{c} * {\left({D}_{c}/{D}_{p}\right)}^{\left(1/3\right)} / {x}_{c}$

Therefore, the maximum density difference between the core and the fluid can be given by substituting the above expression to get the maximum density difference between the core and the fluid ρ*_{c}-*p*_{f}* or Δ*ρ_{cf}*: $\left|{ρ}_{p}-{ρ}_{f}\right| \leq 18750 / {{D}_{p}}^{2}$ $\left|{ρ}_{c}*{\left({D}_{c}/{D}_{p}\right)}^{\left(1/3\right)}/{x}_{c}-{ρ}_{f}\right| \leq 18750 / {{D}_{p}}^{2}$

Therefore: ${ρ}_{c} \leq \left({ρ}_{f}+31250/{{D}_{p}}^{2}\right) * {x}_{c} / {\left({D}_{c}/{D}_{p}\right)}^{\left(1/3\right)} , to minimize settling$ ${ρ}_{c} \geq \left({ρ}_{f}-31250/{{D}_{p}}^{2}\right) * {x}_{c} / {\left({D}_{c}/{D}_{p}\right)}^{\left(1/3\right)} , to minimize floating$

Where larger particles are used, core density is critical and low density materials are preferable. Where smaller particles are used, the core density is less critical and higher density materials, such as salts can be used. Low density materials include sugars (*e.g.*, sucrose and sorbitol, carbohydrates *(e.g.,* starch and glycogen), saturated fatty acids (*e.g.,* stearic acid, myristic acid, palmitic acid, and their derivatives, waxes (e.g., polyethylene wax), polymers (*e.g.*, polyvinyl alcohol (PVA), partially-hydrolylzed polyvinyl alcohol (PHPVA), polyethylene glycol (PEG), polyethylene oxide (PEO), polyvinylpyrrolidone (PVP), hydroxypropylmethyl cellulose (HPMC), hydroxypropylmethylcellulose (HPMC), intermediately-hydrolyzed PVA (IHPVA), fully-hydrolyzed PVA (FHPVA), plasticized PVA, carboxymethyl cellulose (CMC), carboxymethyl dextran (CMD), diethylaminoethyl dextran (DEAED), ethylhydroxyethyl cellulose (EHEC), hydroxyethyl cellulose (HEC), hydroxypropyl cellulose (HPC), hydroxyethylmethyl cellose HEMC), hydroxypropyl dextran (HPD) methyl cellulose (MC), polypropylene glycol (PPG), polypropylene oxide (PPO), polyvinylsulfuric acid (PVSA) and alginates, having a molecular weight such that the polymer is solid at room temperature), and combinations, thereof. Higher density materials include salts, such as sodium sulfate.

The core may include fillers, buffers, stabilizers, plasticizers, distintegrants, extenders, lubricants, dyes, pigments, fragrances and the like, but all such components contribute to the density of the core, and must be selected accordingly. The core may include pockets of trapped air or other gases, which lower the density of the core. The core may include enzymes or enzymes may be coated onto a core that either includes or does not include enzymes.

The nominal diameter and size distribution of the particles is not critical but can be tailored to suit manufacturing, performance, safety, and other requirements. Smaller particles having an enzyme/active coating generally have a higher payload to core weight ratio but are more readily aerosolized. Particles smaller than 10 µm, and especially smaller than 5 µm, should be avoided for respiratory tract safety reasons. Particles smaller than about 40 µm are not visible to the human eye. Larger particles, *e.g.*, greater than about 100 µm, 150 µm, or even 200 µm, are visible to the human eye and may be brightly colored such that they are prominently visible in the enzyme suspension. Exemplary size ranges are 50-100 µm, 50-150 µm, 100-150 µm, 100-200 µm, 150-250 µm, 200-250 µm, 200-300 µm, 250-300 µm, 300-350 µm, 300-400 µm, 350-500 µm, 400-550 µm, and the like. In some cases, the size distribution range is narrow, such that the particles are uniform in size. In some cases, the size distribution is not critical.

Preferably, the cores dissolve or disperse in water within 15 min, 10 min, 5 min, 3 min, 2 min, or even 1, min following the dilution of the low-water liquid composition with at least one volume of water. In the case of smaller cores, *e.g.*, less than about 40 µm, which are not visible to the human eye, it is not critical that the cores dissolve during the cleaning application (e.g., laundry cycle) but they are preferably biodegradable such that they do not accumulate in the environment.

### 2. Cores with density modifiers

The overall density of the particles can also be modified by the incorporation of density modifiers. Density modifiers can be included in the core, itself, or provided in a coating layer. Density modifiers can be included in the core, itself, or provided in an enzyme/active-layer or coating layer. An advantage of providing the density modifier in an enzyme/active-layer or coating layer is that a preselected core can be fine-tuned for use in a given low-water composition simply by varying the amount of density modifier in a subsequently-applied coating.

Exemplary density modifiers are materials having a density of less than 1 g/cm³, and include starch, cellulose fibers, diatomaceous earth, feather particles, zeolites (such as used for molecular sieving), flour, milled plant derived fragments such as corn cobs, soy grit, corn syrup solids, among other small-particle, highly-porous materials. Other acceptable density modifiers include perlite and fumed silica (particularly, fumed silica that has been treated so as to be hydrophobic). It has been found that perlite and starch are especially useful for making roughly spherical low-density granules having a diameter of less than 700 µM via a fluidized-bed spray coating process. Other possible density modifiers include fly ash, borosilicate glass hollow spheres, fused glass hollowspheres, ceramic hollowspheres, plastic hollowspheres, hollow fibers (e.g., DACRON^{®} (DuPont)), low density forms of silicates (such as sodium aluminosilicates used as flow aids for powders), low density forms of silicon dioxide (such as those used as flow aids for powders), sawdust, and/or aerogel shards.

### 3. Properties of particles with low-density cores

Low-density particles are defined by the formulae provided above. In some embodiments, the particles have an overall true density *(i.e.,* the mass of a particle divided by its volume, excluding open pores and closed pores) of less than 1.6 g/cm³, less than 1.5 g/cm³, less than 1.4 g/cm³, less than 1.3 g/cm³, or even less than 1.2 g/cm³, for example, 1.0-1.6 g/cm³, 1.0-1.5 g/cm³, 1.0-1.4 g/cm³, 1.0-1.3 g/cm³, and 1.0-1.2 g/cm³, and the difference between the overall true density of the particles and the density the low-water liquid composition in which they are intended to be suspended is less than ±0.5 g/cm³, less than ±0.4 g/cm³, less than ±0.3 g/cm³, less than ±0.2 g/cm³, even less than ±0.1 g/cm³ or even less than ±0.05 g/cm³. This allows the particles to remain substantially suspended in the liquid composition without falling out of suspension, as is typical of conventional particles. True density can be calculated as described in Example 3. As mentioned, above, the particles can be sufficiently large to be visible to the human eye, e.g., to compliment the appearance of the low-water composition in which they are intended to be dissolved, or can sufficiently small to be invisible to the human eye. Where the particles are intended to be visible, they can include dyes and pigments.

When present in the liquid suspension, enzymes are dissolved at less than 1 gram per liter in the carrier liquid for at least the first 30 days of storage at 25°C, and less than 20% of the enzyme is dissolved within the carrier liquid phase. The enzymes are catalytically active upon dilution of the particles in suspension with at least one volume of water and exhibit most of their original catalytic potential within minutes of dilution. In some embodiments, the enzymes exhibit at least about 50, 60, 70, 80, 90, 95% or essentially all of their original catalytic potential in less than 1, less than 2, less than 3, less than 4, or less than 5 minutes at a preselected temperature.

### III. Preparation of particles

The present particles can be made by methods known to those skilled in the art of particle generation, including but not limited to fluid-bed coating, prilling, spray drying, drum granulation, high shear agglomeration, or combinations of these techniques. Most preferably, the granules are made by a fluidized-bed spray coating process (as exemplified below).

### IV. Compositions containing the liquid enzyme suspensions

The present particles may be included in low-water compositions, such as those used for cleaning, disinfection, decontamination, textile processing, feed, and food. The compositions may 5-20% water by weight. In some embodiments, the composition containing an enzyme suspension contains any of about 5-10%, 10-15%, or 15-20% water by weight (w/w). Exemplary liquid laundry detergent composition in which the particles may be suspended include but are not limited to PUREX^{®} ULTRAPACKS (Henkel), FINISH^{®} QUANTUM (Reckitt Benckiser), CLOROX^{™} 2 PACKS (Clorox), OXICLEAN MAX FORCE POWER PAKS (Church & Dwight), TIDE^{®} STAIN RELEASE, CASCADE^{®} ACTIONPACS, TIDE^{®} and ARIEL^{®} PODS^{™} and GAIN FLINGS (Procter & Gamble), ALL^{™} MIGHTY PACS (Sun Products), KIRKLAND SIGNATURE^{™} ULTRACLEAN PACS^{™}.

Enzyme(s) of interest present in the low-density particles are stable in low-water compositions for at least 9 days at 37°C and are catalytically active upon dilution of the low water compositions with at least one volume of water. In some embodiments, an enzyme of interest is stable in the low water for about 2 weeks, 1 month, 2 months, or 3 months or longer at 25°C and exhibits at least about 50, 60, 70, 80, 90, 95% or essentially all of its initial catalytic potential upon dilution in water.

Where the low water composition is a detergent composition, it may contain one or more surfactants, builders, bleaches, bleach precursors, bleach activators, enzyme stabilizers, complexing agents, chelating agents, foam regulators, corrosion inhibitors, anti-electrostatic agents, dyes, perfumes, bactericides, fungicides, and activators, and any other ingredients typically found in laundry, dishwashing (including automatic and hand dishwashing), and other cleaning compositions.

In some embodiments, the detergent composition does not contain boron or borate. In some embodiments, the detergent contains a low (e.g., submillimolar) level of calcium. In some embodiments, the detergent composition contains low (e.g., submillimolar) levels of period IV metals, *e.g.*, K, Ca, Mn, Fe, Co, Ni, Cu, Zn.

### V. Methods of use

The present particles may be used in any application where enzymatic activity is desired from a low-water liquid composition intended to be diluted prior with at least one volume of water in use. Upon dilution, at least about 50, 60, 70, 80, 90, or 95% of the enzyme is soluble and catalytically active in the diluted composition.

In some embodiments, the application is cleaning and activation is performed in a bucket or other container, including a container to be cleaned. In the case of a laundry detergent composition, activation is typically performed in a washing machine. In the case of a dishwashing detergent composition, activation is typically performed in a dishwasher. In the case of a textile composition, activation is typically performed in a suitable bath. In the case of a food, beverage, or feed, activation is performed where needed to deliver active enzyme to the site of application.

The particles are particularly useful as components of a cleaning composition, such as a detergent composition, *e.g.,* a laundry detergent composition or a dishwashing detergent composition. Especially preferred is a liquid laundry detergent composition. Such cleaning compositions typically comprise a cleaning adjunct, or preferably a combination of cleaning adjuncts. Typically, the cleaning adjunct will be present in the composition in an amount from 0.001 to 99.9 wt%, more typically from 0.01 to 80 wt% cleaning adjunct. An exemplary formulation with suitable cleaning adjuncts in the form of a unit dose laundry detergent composition is provided, below. Such a unit dose formulations can comprise one, two three or more compartments. The components in each compartment may be different or the same, but the overall/total ingredients of the unit dose formulation have the same composition.

The following examples are intended to illustrate, but not limit, the low-density particles.

### EXAMPLES

### Example 1

### Evaluation of Particle Agglomeration in Low-Water Detergent Compositions

10 g of laundry detergent was added to a clear 15 ml test tube. Approximately 0.2 g of particles was added and mixed to form a well-dispersed suspension. The tubes were placed on an end-over-end mixer and rotated at low RPM at room temperature *(i.e.,* 25°C), representing nominal movement under manufacturing and storage conditions. The degree of agglomeration was visually assessed after 7 days. An ideal result was that all particles remain as individual particles that are not associated with any other particle. Less ideal results include the observation of groupings of small numbers of particles. The least ideal results are the agglomeration of tens to hundreds of particles, or more, together.

### Example 2

### N-succinyl-L-alanyl-L-alanyl-L-prolyl-L-phenyl-p-nitroanilide (AAPF-pNA) assay to measure protease activity

The following reagent solutions were used:
AAPF substrate stock: 160 mM *(i.e.,* 100 mg/mL) suc-AAPF-pNA dissolved in dimethylsulfoxide (DMSO), Stability buffer: 100 mM MES (pH 5.5) with 0.005% v/v Tween 80 (may optionally include 10 mM CaCl₂), Activity buffer:100 mM Tris (pH 8.5 or 8.6) with 0.005% v/v Tween-80 (may optionally include 10 mM CaCl₂), Assay solution (substrate stock diluted 1: 100 into activity buffe)r: 1.6 mM AAPF-pNA in 100 m1M Tris (pH 8.5 or 8.6).

Procedure: An enzyme standard curve was prepared by making serial dilutions of purified subtilisin protease (0.5-10 ppm) in stability buffer. Test samples were prepared to achieve protease concentrations between 1-10ppm in stability buffer. Assay solution was prepared by diluting the substrate stock 1:100 with activity buffer. 200 µL of assay solution was added to each well of a 96-well plate.

The assay was performed by adding 10 µl of diluted protease enzyme solution to each well of the assay solution plate. The solutions were mixed for 10 seconds, and the absorbance change was measured at 410 nm in a microplate reader at 25°C (set in kinetic mode, over 2 minutes). The subtilisin protease activity (AU = activity units) was calculated as mOD₄₁₅/min x dilution factor, where mOD₄₁₀ refers to the optical density of the reaction product multiplied times 1000 as measured at 410 nm.

### Example 3

### Making and Testing Particles

Various particles were made using standard fluid bed methods, as exemplified in US6413749, which is incorporated by reference. The particle types, labeled A-C, are summarized in Table 2. The composition of the core (Core), a first coating layer containing the enzyme(s) with or without a binder and/or with or without a density modifier (SP1), second coating layer (SP2), third coating layer (SP3), as applicable, are indicated. All particles included the indicated amount of a variant subtilisin protease (enz), which allowed protein release and leakage to be measure using a standardized protease activity assay as described in Example 2

**Table 2. Description of particles**

| **Particle** | **Core** | **SP1** | **SP2** | **SP3** |
|---|---|---|---|---|
| A | Mini sucrose | 22.5% enz, | 5% PVA, 4%TiO₂, | 2% PVA |
| | | 2% PVA 10% starch | 1% Neodal | 1% Flexiverse Green |
| B | Mini sucrose | 22.5% enz, | 5% PVA, 4%TiO₂, | 2% Carnauba Wax |
| | | 2% PVA 10% Starch | 1% Neodal | 1% Flexiverse Green |
| C | Mini sucrose | 22.5% enz, | 6% PVA, 4%Talc, | |
| | | 2% PVA | 2% Carnauba Wax | |
| | | 10% Starch | 1% Flexiverse Green | |

The particles were tested for agglomeration performance criteria in low water laundry detergents. Particle B remained as individual particles suspended in the detergent after the evaluation period, whereas particle A had significant agglomeration with tens to hundreds of particles agglomerated together and no longer suspended in the detergent. Particles C are expected to have intermediate properties.

All publications, patents, and patent applications cited herein are hereby incorporated by reference in their entireties for all purposes and to the same extent as if each individual publication, patent, or patent application were specifically and individually indicated to be so incorporated by reference.

The following numbered paragraphs (paras.) contain further statements of various aspects and embodiments of the present invention:-
1. Particles capable of isolating and stabilizing enzymes in a liquid composition without agglomerating in manufacturing and/or storage, comprising:
   (a) a core, including an active component, and/or a core having a first coated layer comprising an active component immediately deposited upon the core; and
   (b) an outer-most coated layer comprising a hydrophobic, water-insoluble, water-disintegrating-material having an amount of water solubility of less than about 1 mg/mL in water at 25°C:
      wherein the coated layer in (b) fully disintegrates within about 5 minutes when the liquid composition is diluted 1: 1 with water at 25°, allowing the dissolution of the enzyme and/or active component into the diluted liquid composition, and
      wherein the particles exhibit reduced aggregation in the liquid composition compared to otherwise identical particles comprising a third coated layer comprising a water-soluble polymer having a solubility of greater than about 1 mg/mL in water at 25°C.
2. The particles of para 1, comprising, between (a) and (b) at least one additional layer comprising a water-soluble polymer and an active ingredient.
3. The particles of para 1, comprising, between (a) and (b) at least one additional layer comprising a water-soluble polymer lacking an active ingredient.
4. The particles of para 1 or 2, wherein the core lacks an active component.
5. The particles of para 1 or 3, wherein the core includes an active component.
6. The particles of any of the preceding paras , wherein the outer-most coating disintegrates within 5 minutes, within 4 minutes, within 3 minutes, within 2 minutes, within 1 minute, within 30 seconds, or even within 15 seconds after a liquid composition containing the particles is contacted with at least one additional volume of water at 25°C.
7. The particles of any of the preceding paras , wherein the outer-most coating represents less than 8%, less than 7%, less than 6%, or even less than 5% of the overall weight of the particle.
8. The particles of any of the preceding paras , wherein the outer-most consists essentially of, or consists of, a hydrophobic, water-insoluble, water-disintegrating-material having an amount of water solubility of less than about 1 mg/mL in water at 25°C.
9. The particles of any of the preceding paras , wherein the core has a density defined by the equations: ${ρ}_{c} \leq \left({ρ}_{f}+31250/{{D}_{p}}^{2}\right) * {x}_{c} / {\left({D}_{c}/{D}_{p}\right)}^{\left(1/3\right)}$ and ${ρ}_{c} \geq \left({ρ}_{f}-31250/{{D}_{p}}^{2}\right) * {x}_{c} / {\left({D}_{c}/{D}_{p}\right)}^{\left(1/3\right)} ,$ wherein ρ*_{c}* is the density of the core in in g/cm³, *p_{f}* is the mass density of the liquid composition in g/cm³, x*_{c}* is the mass fraction of the core in the particle, D*_{c}* is the diameter of the core in µM, and D*ₚ* is the diameter of the particle in µM.
10. The particle of any of the preceding paras, having an overall true density of less than 1.6 mg/mL, less than 1.4 mg/mL, or even less than 1.2 mg/mL.
11. A method for reducing the agglomeration of particles in manufacturing and/or storage, comprising coating the particles in an outer-most layer comprising a hydrophobic, water-insoluble, water-disintegrating-material having an amount of water solubility of less than about 1 mg/mL in water at 25°C.
12. The method of para 11, wherein the outer-most consists essentially of, or consists of, a hydrophobic, water-insoluble, water-disintegrating-material having an amount of water solubility of less than about 1 mg/mL in water at 25°C.
13. The method of para 11 or 12, wherein the outer-most coating disintegrates within 5 minutes, within 4 minutes, within 3 minutes, within 2 minutes, within 1 minute, within 30 seconds, or even within 15 seconds after a liquid composition containing the particles is contacted with at least one additional volume of water at 25°C.
14. The method of any of para 11-13, wherein the outer-most coating represents less than 8%, less than 7%, less than 6%, or even less than 5% of the overall weight of the particle.

## Claims

1. A method for reducing the agglomeration of particles in manufacturing and/or storage, comprising coating the particles in an outer-most layer comprising a hydrophobic, water-insoluble, water-disintegrating-material having an amount of water solubility of less than about 1 mg/mL in water at 25°C.

2. The method of claim 1, wherein the outer-most consists essentially of, or consists of, a hydrophobic, water-insoluble, water-disintegrating-material having an amount of water solubility of less than about 1 mg/mL in water at 25°C.

3. The method of claim 1 or 2, wherein the outer-most coating disintegrates within 5 minutes, within 4 minutes, within 3 minutes, within 2 minutes, within 1 minute, within 30 seconds, or even within 15 seconds after a liquid composition containing the particles is contacted with at least one additional volume of water at 25°C.

4. The method of any of claims 1-3, wherein the outer-most coating represents less than 8%, less than 7%, less than 6%, or even less than 5% of the overall weight of the particle.

5. The method of any of claims 1-4, wherein the particles comprise a core including an active component and/or a core having a first coated layer comprising an active component immediately deposited upon the core.

6. The method of claim 5 wherein the particles comprise at least one additional layer between the core and the outer-most layer comprising a water-soluble polymer and an active ingredient.

7. The method of claim 5 wherein the particles comprise at least one additional layer between the core and the outer-most layer comprising a water-soluble polymer lacking an active ingredient.

8. The method of claims 5 or 6 wherein the core lacks an active component.

9. The method of claims 5 or 7 wherein the core includes an active component.

10. The method of any of the preceding claims, wherein the core has a density defined by the equations: ${ρ}_{c} \leq \left({ρ}_{f}+31250/{{D}_{p}}^{2}\right) * {x}_{c} / {\left({D}_{c}/{D}_{p}\right)}^{\left(1/3\right)}$ and ${ρ}_{c} \geq \left({ρ}_{f}-31250/{{D}_{p}}^{2}\right) * {x}_{c} / {\left({D}_{c}/{D}_{p}\right)}^{\left(1/3\right)} ,$ wherein ρ*_{c}* is the density of the core in in g/cm³, *p_{f}* is the mass density of the liquid composition in g/cm³, x*_{c}* is the mass fraction of the core in the particle, D*_{c}* is the diameter of the core in µM, and D*ₚ* is the diameter of the particle in µM.

11. The method of any of the preceding claims, wherein the particle has an overall true density of less than 1.6 mg/mL, less than 1.4 mg/mL, or even less than 1.2 mg/mL.
